# EUROPÄISCHE PATENTSCHRIFT

(11) **EP 1 143 883 B1**
(45) Veröffentlichungstag und Bekanntmachung des Hinweises auf die Patenterteilung: **19.10.2005**
(21) Anmeldenummer: 00951181.7
(22) Anmeldetag: 23.08.2000
(51) Int. Cl.: A61F 2/30

(54) **FEMURKOPF-SEGMENTPROTHESE ZUM PRÄZISEN EINBAU DER SEGMENTPROTHESE**
FEMUR HEAD-SEGMENT PROSTHESIS FOR PRECISE INSERTION OF A SEGMENT PROSTHESIS
PROTHESE PARTIELLE DE TETE FEMORALE POUR IMPLANTER CETTE PROTHESE AVEC PRECISION

(30) Priorität: 23.08.1999 CH 152299
(43) Veröffentlichungstag der Anmeldung: 17.10.2001
(73) Patentinhaber: Gautier, Emanuel, 1708 Fribourg (CH)
(72) Erfinder: Gautier, Emanuel, 1708 Fribourg (CH)
(86) Internationale Anmeldenummer: PCT/CH2000/000447
(87) Internationale Veröffentlichungsnummer: WO 2001/013823

(56) Entgegenhaltungen:
- EP-A- 0 485 326
- EP-A- 0 505 634
- EP-A- 0 773 007
- WO-A-98/07393
- FR-A- 2 578 739
- FR-A- 2 728 157

## Beschreibung

Die Erfindung liegt im Gebiete der Medizinaltechnik und betrifft Prothesenkomponenten zum Einbau nach den Oberbegriffen der entsprechenden Patentansprüche. Die Implantate und Verfahren dienen dem prothetischen Ersatz eines Teils der Femurkopfoberfläche bei degenerativer, traumatischer oder vaskulärer Schädigung der Knorpeloberfläche, respektive des subchondralen Knochens.

Schädigungen oder Defekte der Knorpeloberfläche von Gelenken werden gemäss dem Stand der Technik entweder repariert (Allotransplantat, Autotransplantat, Transplantation von Knorpelgewebe aus Chondrozytenzucht), aus der Belastungszone weggedreht (intertrochantere Osteotomie, Beinachsenkorrektur) oder durch eine Prothese oder Teilprothese ersetzt. Im Bereich des Hüftgelenkes ist die Totalprothese mit Ersatz der azetabulären und femoralen Knorpeloberfläche durch entsprechend ausgeformte prothetische Implantate Therapie der Wahl bei fortgeschrittenen degenerativen Erkrankungen und höherem Lebensalter eines Patienten. Typischerweise wird dabei auf der femoralen Seite der ganze Hüftkopf entfernt und ein Prothesenstiel als Träger des Prothesenkopfes in den Femurschaft einzementiert oder zementfrei verklemmt. Auf der Femurseite wird dabei der ursprünglichen Knochenstocks weitgehend geopfert, um die Prothese in den Femurschaft einbringen zu können. Bei einem neueren alternativen Prothesendesign, der Druckscheibenprothese nach Huggler (Sulzer Orthopedics Winterthur), wird der Schenkelhals hoch (proximal) reseziert, die Femurkomponente direkt auf den Schenkelhalsstumpf aufgesetzt und über eine Schenkelhalsschraube auf eine laterale Seitenplatte stabilisiert, dies mit dem Ziel, den Knochenstock femoral bestmöglich zu erhalten.

Bei jüngeren Patienten wird die Knorpeloberfläche des Hüftkopfes entweder durch ein Trauma (Hüftgelenkluxationsfraktur) direkt partiell zerstört oder durch eine vaskuläre Störung im Bereich des subchondralen Knochens mit konsekutiver Osteolyse seines mechanischen Tragsystems beraubt. Im Falle einer sogenannten avaskulären Femurkopfnekrose führt die Impaktion der Gelenkoberfläche im Stadium III zur Inkongruenz und damit zwangsläufig zum Entstehen einer Coxarthrose. Der die Arthrose verursachende Faktor liegt dabei primär im Bereich des femoralen Gelenkanteils, währenddem der azetabuläre Anteil primär immer intakt ist und erst sekundär durch die Entrundung des femoralen Gelenkpartners geschädigt wird.

Die Standzeit einer herkömmlichen Hüfttotalprothese ist beim jüngeren Patienten eher kurz, da es durch Abriebpartikel der Prothesenkomponenten zur Osteolyse und damit zur Auslockerung der Prothesenkomponenten kommt. Die teils ausgedehnten periprothetischen Osteolysen bereiten im Falle von wiederholt notwendigen Prothesenwechseloperationen zunehmend technische Probleme bei der erneuten Prothesenverankerung. Aus chirurgischer Sicht ergibt sich damit ein hohes Interesse, beim jungen Patienten mit einer auf den Femurkopf beschränkten Gelenkschädigung den femoralen Knochen soweit wie möglich zu erhalten und die geschädigte, defiziente oder impaktierte Knorpeloberfläche des Femurkopfes möglich minimal durch eine femorale Teilprothese zu ersetzen. *Eine derartige Femurkopf Teilprothese ist als zementiertes Monobloc-Implantat bereits auf dem Markt erhältlich, hergestellt beispielsweise von Tomier, Ismier France.*

Es zeigt sich, dass der Erfolg einer Teilprothese des Femurkopfes in hohem Masse abhängig ist von der Präzision der Positionierung der Prothesenkomponten und damit von der exakten Wiederherstellung der Gelenkkongruenz. Qualität und Langlebigkeit der primär mechanischen und sekundär biologischen Verankerung der Prothese, Materialbeschaffenheit der artikulierenden Prothesenoberfläche mit möglichst niedrigem Reibungskoeffizienten gegenüber dem azetabulären Knorpelüberzug und optimal geringes Abriebverhalten der Prothesenoberfläche und des erhaltenen Gelenkknorpels sind weitere Elemente eines erfolgversprechenden Prothesen-Designs.

Die Erfindung stellt sich aus diesem Grunde die Aufgabe, Prothesenkomponenten zum prothetischen Teilersatz des Hüftkopfes zu entwickeln oder bestehende prothetische Implantate und Verfahren zu verbessern, um die Erfolgschancen derartiger operativer Eingriffe am Hüftgelenk jüngerer Patienten unter maximal möglichem Belassen des Knochenstocks zu erhöhen, respektive den sinnvollen prothetischen Teilersatz des Hüftkopfes erst zu ermöglichen. Die erfindungsgemässen Implantate sollen die prothetische Oberflächenrekonstruktion am Hüftgelenk mit grösstmöglicher Präzision erlauben. Die erfindungsgemässen Prothesenkomponenten sollen modular aufgebaut sein, einfach herstellbar und insbesondere einfach handhabbar sein.

Diese Aufgabe wird gelöst durch Prothesenkomponenten, wie sie im unabhängigen Patentanspruch definiert ist. Die abhängigen Patentansprüche definieren weitere Ausführungsformen. Die Lösung der erwähnten generellen Aufgabe wird durch die Entwicklung, Neuerfindung oder Verbesserung im Bereich der untenstehend beschriebenen Teilaspekte erreicht. Diese Teilaspekte sind bezüglich ihrer Wirksamkeit zum Erreichen des gesteckten Ziels nicht gleichwertig, sie können demzufolge unterschiedliche Wirkung entwickeln und können je nach ihrer Wertigkeit auch weggelassen werden. Die Hauptaspekte der neuen modularen Hüftkopf-Segmentprothese sind die folgenden:
- Die vorgeschlagene Segmentprothese zum Teilersatz des Femurkopfes (sowie eines Teils seiner knorpeligen Oberfläche) ist modular aufgebaut und besteht aus einer Trägerkomponente, einer Prothesenkalotte und je nach Einbaugenauigkeit mindestens einem zwischen Trägerkomponente und Prothesenkalotte eingebrachten doppelt konischen Korrekturring. Die Modularität der Prothese wird durch konische Steckverbindungen zwischen den einzelnen Prothesenkomponenten gewährleistet. *Konische Zwischenringe sind zur genauen Ausrichtung von Prothesenkomponenten dem Stand der Technik gemäss bekannt und patentiert. Die Ausrichtung eines Protheseninlays in einem metallischen Prothesencup mittels derartiger konischer Zwischenringe zur Anpassung von Inklination und Anteversion der artikulierenden Prothesenoberfläche wird im Europäischen Patent EP-A-0 773 007 beschrieben. Eine entsprechende technische Ausführungsform wird bei der vorgeschlagenen Segmentprothese des Femurkopfs zur passgenauen Ausrichtung der Prothesenkalotte an die erhaltene Knorpeloberfläche des Femurkopfs eingesetzt*.
- Alle Komponenten sind in zueinander passenden, unterschiedlichen Dimensionen gefertigt, um das Spektrum unterschiedlicher Femurkopfdurchmesser im Bereich zwischen 38 und 60 Millimetern abdecken zu können.
- Die Trägerkomponente wird in zwei unterschiedlichen Ausformungen hergestellt, die eine dient der unzementierten Implantation, die andere der zementierten Implantation.
- Bei der unzementierten Implantation wird der spongiöse Knochen durch das Einpressen der aussen konisch ausgeformten Trägerkomponente radiär so vorgespannt, dass eine hohe Primärstabilität erreicht wird. Diese Primärstabilität kann im Bedarfsfall durch das Einsetzen von sich im Boden der Trägerkomponente winkelstabil verblockenden Schrauben oder Stiften verbessert werden. Als alternative unzementierte Fixation kann im Bereich der Aussenseite der Trägerkomponente ein selbstschneidendes gleichsinnig verlaufendes Zweifach- oder Dreifachgewinde zur Anwendung kommen.
- Für die unzementierte Implantation besteht die Trägerkomponente aus Titan oder einer Titanlegierung. Seine Aussenfläche ist rauh (sandgestrahlt), mit Titanplasma beschichtet oder mit einem Titangitternetz versehen, was eine hohe Sekundärstabilität durch Osteointegration zur Folge hat.
- Bei der zementierten Implantation wird eine Trägerkomponente von generell gleicher Ausformung und Dimensionierung wie für die unzementierte Implantation verwendet. Die Trägerkomponente besteht aus einem rostfreien Stahl. Die Aussenfläche ist glatt, der Boden weist keine zusätzlichen Bohrungen für eine allfällige Verschraubung auf.
- Die Prothesenkalotte weist einen Öffnungswinkel von zwischen 90° und 120° auf. Sie ist in jeweils um einen Millimeter in seinem Durchmesser wachsender Grösse zwischen 38 mm und 60 mm gefertigt. Sie besteht aus einem Metall, einer Metall-Legierung, einem keramischen Material oder einem Kunststoff. Die Prothesenkalotte weist einen nach aussen weisenden Konus zur Verbindung mit den anderen Prothesenbestandteilen auf. Die Wahl des Materials ergibt sich durch Optimierung der Reibungsverhältnisse zum intakten Knorpel, der Benetzbarkeit und des Abriebverhaltens.
- Die Prothesenkalotte kann in veränderter Form und mit verändertem Material (Titan, Titanlegierung) alternativ auch als Trägerkomponente für eine biologische Oberfläche zum Beispiel für eine im Labor gezüchtete Knorpelzell- oder -gewebeschicht dienen. In diesem Falle wird die Prothesenkalotte direkt als Oberfläche für die Knorpelzucht verwendet und später zusammen mit dem Knorpelgewebe als teils biologische Prothesenkalotte implantiert.
- Die Korrekturringe sind aus Titan oder rostfreiem Stahl gefertigt. Sie sind innen und aussen konisch ausgeformt, die Konus-Oberfläche ist glatt. Das Einsetzen unterschiedlicher Zwischenringe erlaubt eine Korrektur einer in Höhe oder Angulation unpräzise eingesetzten Trägerkomponente.

Im Zusammenhang mit den nachfolgenden Figuren sollen die Problematik der partiellen Destruktion der Knorpeloberfläche und des subchondralen Knochens am Beispiel der avaskulären Femurkopfnekrose sowie die Prothesenkomponenten und Verfahren zum prothetischen Teilersatz des Hüftkopfs im Detail beschrieben werden. Dabei zeigen:
- **Figur 1**: eine Übersicht des modularen Prothesensystem für den Teilersatz des Femurkopfes;
- **Figur 2**: eine schematische Darstellung der konischen Trägerkomponente der modularen Segmentprothese;
- **Figur 3**: eine schematische Darstellung eines ersten doppelt konischen Korrekturringes zur korrekten axialen Ausrichtung der Prothesenkalotte bei desaxiertem Einbau der Trägerkomponente der modularen Segmentprothese;
- **Figur 4**: eine schematische Darstellung eines zweiten doppelt konischen Korrekturringes zur korrekten Höhenausrichtung der Prothesenkalotte bei zu hohem (proximalen) oder zu tiefem (distalen) Einbau der Trägerkomponente der modularen Segmentprothese;
- **Figur 5**: eine beispielhafte Darstellung des Einbaus der Trägerkomponente in korrekter Neutralposition, in zu hoher (proximaler) und zu tiefer (distaler) Lage sowie die entsprechende Höhenkorrektur der Prothesenkalotte mit Hilfe des zweiten doppelt konischen Korrekturring-Systems;
- **Figur 6**: eine schematische Darstellung unterschiedlicher Durchmesser des ursprünglichen Femurkopfes (38 - 60 mm) und die entsprechend notwendigen unterschiedlichen Grössen von Trägerkomponente, Prothesenkalotte mit Positivkonus und doppelt konischen Korrekturringen;

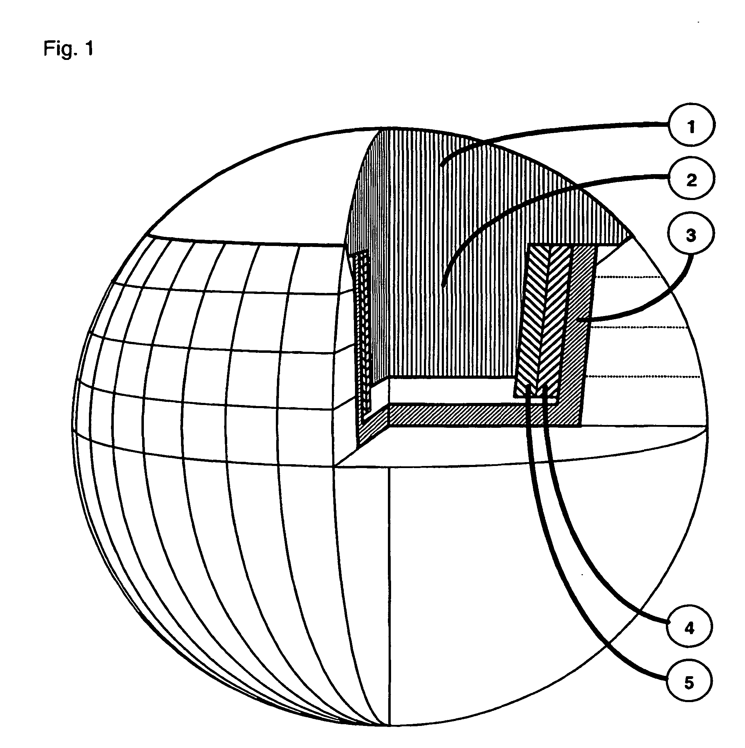

**Figur 1** zeigt schematisch die modular aufgebaute Segmentprothese zum Teilersatz des Femurkopfs. Die Prothese besteht aus einer Prothesenkatotte ***1*** mit positivem (nach aussen weisenden) Verbindungskonus ***2,*** einer Trägerkomponente ***3*** und je nach Fertigung einem oder zwei doppelt konischen Korrekturringen ***4, 5*****.** Der Kalottenteil ***1*** besteht aus Metall oder einem keramischen Material mit niedrigem Reibungskoeffizienten gegenüber Knorpel und guter Benetzbarkeit, er artikuliert entweder direkt mit dem Knorpelüberzug der azetabulären Seite oder er dient alternativ als Träger für eine in vitro künstlich hergestellte Knorpelzell- respektive Knorpelgewebeschicht. Der Kalottenteil ***1*** trägt nach kaudal einen positiven, d.h. nach aussen weisenden Konus ***2*** mit einem Konuswinkel um 5° zur modularen Verbindung mit den Korrekturringen ***4, 5,*** respektive der Trägerkomponente ***3.*** Die an Innen- und Aussenseite konisch ausgeformte Trägerkomponente ***3*** besteht aus Titan oder einer Titanlegierung mit einer rauhen äusseren Oberfläche zur Verbesserung der Osteointegration. Die glatte Innenseite dient der passgenauen Aufnahme von doppelt konischen Korrekturringen ***4, 5.***

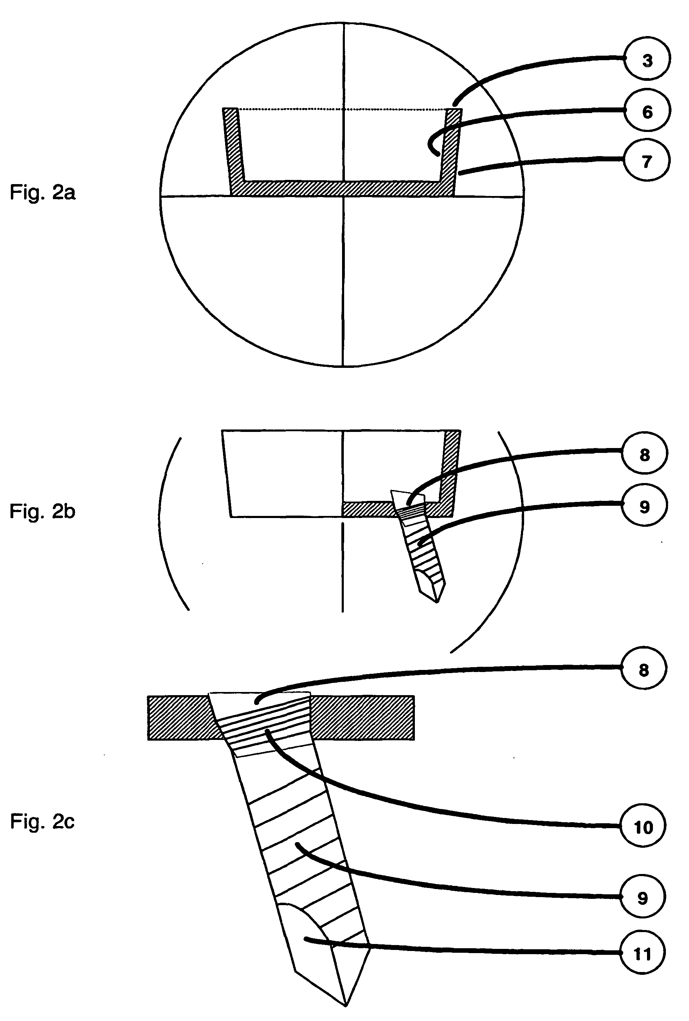

**Figur 2a-c** zeigt den Sitz und die Verankerung der Trägerkomponente ***3.*** Die Trägerkomponente ***3*** ist doppelt konisch ausgeformt mit einem Konuswinkel von zwischen 5° und 40° aussen und von zwischen 5° und 10° innen. Der Aussenkonus ***7*** dient dem satten Einpressen der Trägerkomponente mittels eines speziellen Prothesen-Setzinstrumentes und erzeugt eine radiale Vorspannung im umgebenden spongiösen Knochenlager, dessen Grösse durch den Konuswinkel einerseits und die am Setzinstrument applizierte Einpresskraft andrerseits definiert wird. Seine äussere Oberfläche ist rauh, um die Osteointegration und damit die spätere biologische Sekundärverankerung zu ermöglichen. Der Innenkonus ***6*** weist eine glatte Oberfläche auf und dient der Verbindung mit den Korrekturringen. Zur zusätzlichen Verankerung des Trägerteils ***3*** kommen selbstbohrende und selbstschneidende ***11,*** sich in der Trägerkomponente ***3*** winkelstabil verblockende ***8*** Titanschrauben ***9*** zur Anwendung. Diese Schrauben werden 10 - 20° divergent eingesetzt und haben eine in Abhängigkeit vom Aussendurchmesser des Femurkopfs definierte Länge. Die winkelstabile Verblockung ***8*** der Schrauben ***9*** in der Trägerkomponente ***3*** wird entweder durch einen konisch ausgeformten Schraubenkopf ***10*** oder durch ein konisches Schraubengewinde im Bereich der Schraubenkopf-Aussenseite ***10*** mit entsprechendem Gegengewinde im Bereich des Bodens der Trägerkomponente erreicht. In diesem Falle ist die Gewindesteigung im Bereich des Kopfteils der Schraube kleiner zu wählen als die des Gewindes im Bereich des Schraubenschaftes 9. Alternativ trägt die Trägerkomponente ein an der Oberfläche des Aussenkonus ein doppeltes Verankerungsgewinde, welches das direkte Einschrauben der Trägerkomponente in den spongiösen Knochen erlaubt.

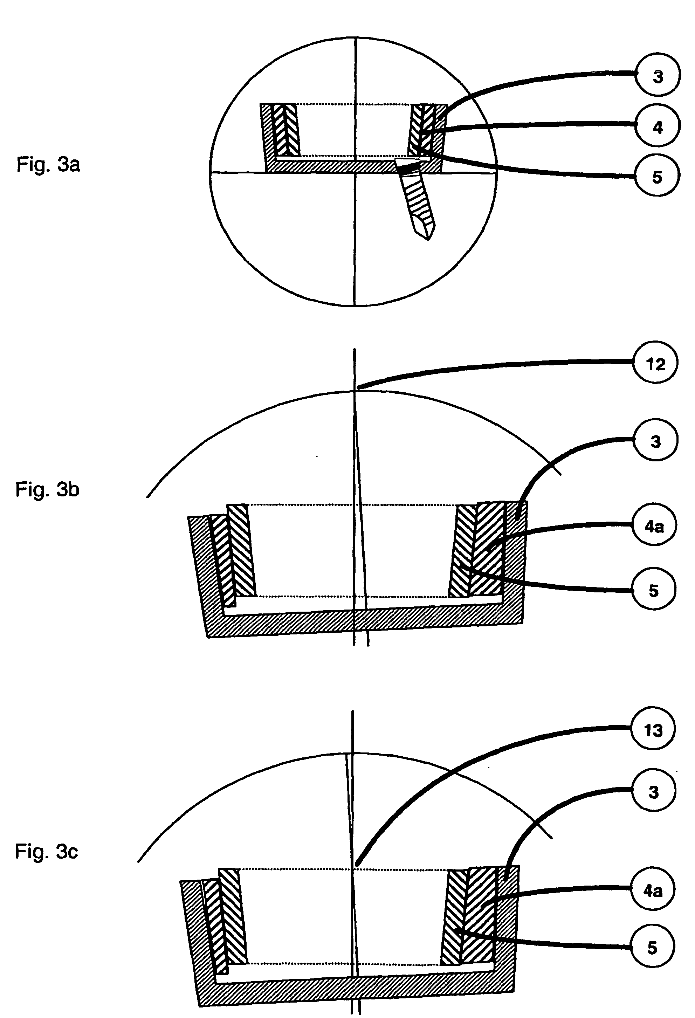

**Figur 3a-c** zeigt beispielhaft die Funktion und die Ausformung des ersten äusseren doppelt konischen Korrekturringes (Winkelkorrektur) ***4.*** Der äussere Korrekturring ***4*** weist sowohl innen als auch aussen eine konische Ausformung auf mit einem Konuswinkel um 5°. Die Innenfläche und die Aussenfläche sind glatt, der Aussenkonus dient der Verbindung mit der Trägerkomponente ***3,*** der Innenkonus dient der Verbindung mit dem zweiten konischen Korrekturring ***5*****.** Der Aussenkonus ist immer koaxial und konzentrisch zur Längsachse der Trägerkomponente ausgerichtet. Der Innenkonus ist gegenüber dem Aussenkonus exzentrisch und anguliert gefertigt, dies in Abhängigkeit von der Notwendigkeit der Achsenkorrektur der Prothesenkalotte. Diese kann notwendig werden bei akzidentell schräg verankerter Trägerkomponente ***3*** der modularen Segmentprothese. Zum präzisen Einpassen der Prothesenkalotte ***1*** sind unterschiedliche Exzentrizitäten und Angulationen dieses Innenkonus von zwischen 0° und 3° ***4a*** notwendig. Der Schnittpunkt zwischen der Längsachse des exzentrisch und anguliert gefertigten Innenkonus des ersten Korrekturrings und der zentrischen Längsachse des Aussenkonus dieses ersten Korrekturrings liegt auf der Oberfläche der Prothesenkalotte ***12*** oder am Oberrand der Trägerkomponente ***13.***

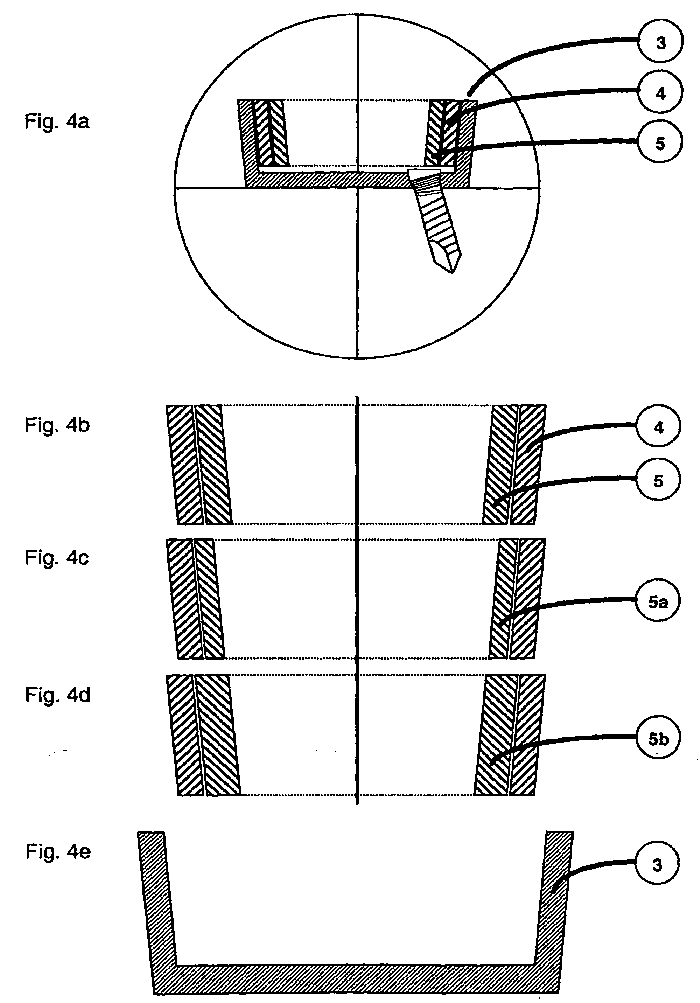

**Figur 4a-e** zeigt schematisch die Funktion und die Ausformung des zweiten inneren doppelt konischen Korrekturringes (Höhenkorrektur) ***5***. Der innere Korrekturring ***5*** weist sowohl innen als auch aussen eine konische Ausformung auf mit einem Konuswinkel um 5°. Die Innenfläche und die Aussenfläche sind glatt, der Aussenkonus dient der Verbindung mit dem äusseren Korrekturring ***4,*** der Innenkonus dient der Verbindung mit dem nach aussen weisenden Verbindungskonus der Prothesenkalotte ***2*** der modularen Segmentprothese. Aussen- und Innenkonus sind zueinander koaxial und konzentrisch gefertigt. Die Wandstärke dieses konischen Korrekturrings ***5*** variiert ***5a, 5b*** und lässt damit korrespondierende Höhenkorrekturen im Bereich von -2 mm bis + 2 mm zu. Technisch lässt sich die Funktion des äusseren Winkel-Korrekturringes und des inneren Höhen-Korrekturringes alternativ auch in einem einzigen doppelt konischen Korrekturring fertigen und zusammenfassen. Dies ergibt aber als Nachteil eine grössere Anzahl Korrekturringe. Die Lösung mit **zwei** Konusringen benötigt **9** Korrekturringe: **5** Höhen-Korrekturringe (-2 mm, -1 mm, 0 mm, +1 mm, +2 mm) und **4** Winkel-Korrekturringe (0°, 1°, 2°, 3°). Die Lösung mit einem kombinierten Höhen- und Winkel-Korrrekturring braucht **20** Korrekturringe: **5** Höhenmal **4** Winkelkorrekturen.

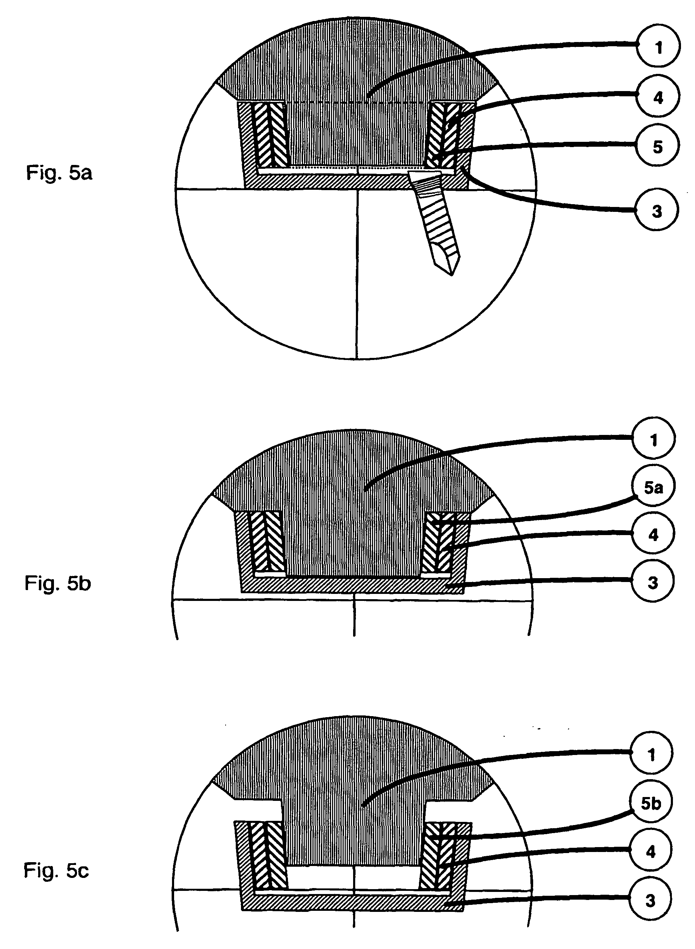

**Figur 5a-c** zeigt schematisch und beispielhaft die Funktion verschiedener Höhen-Korrekturringe ***5.*** Korrekturringe mit verkleinerter Wandstärke ***5a*** führen zu einem tieferen Einsinken der Prothesenkalotte ***1*** und korrigieren damit einen zu proximalen Einbau der Trägerkomponente ***3*** (Figur 5b). Umgekehrt führt eine vergrösserte Wandstärke ***5b*** zu einem weniger tiefen Einsinken der Prothesenkalotte ***1*** und damit zur Korrektur einer zu distal eingebauten Trägerkomponente ***3*** (Figur 5c).

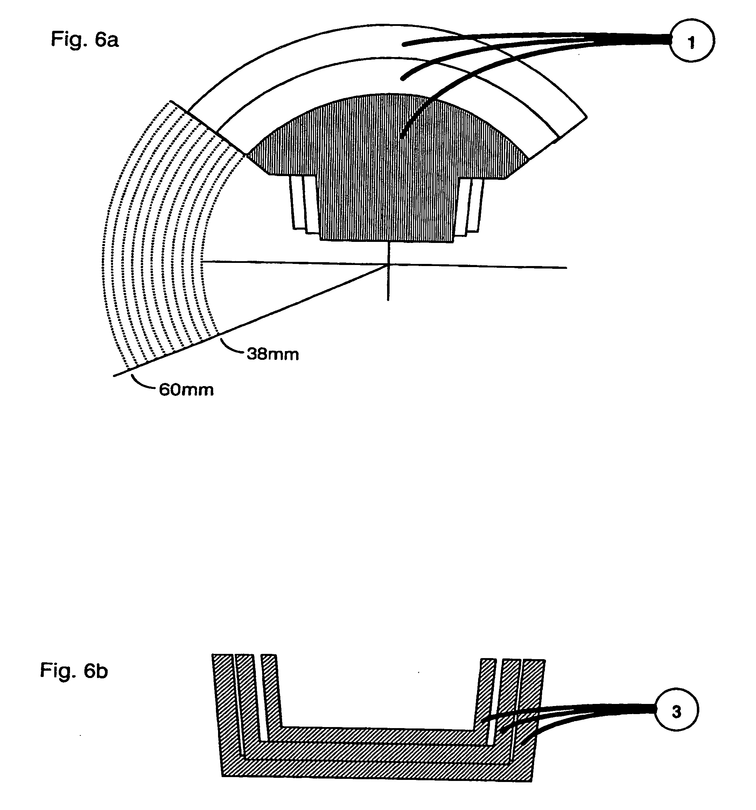

**Figur 6a, b** zeigt schematisch Femurkopfdurchmesser zwischen 38 und 60 mm. Zum präzisen prothetischen Ersatz eines Femurkopfteils braucht es eine feine Abstufung des Durchmessers der Prothesenkalotte von 1 mm. Die Prothesenkalotte soll einen Kugelausschnitt, respektive Kugelabschnitt mit einem Öffnungswinkel von minimal 90° und maximal 120° ersetzen können. Zur optimalen Abstützung der Prothesenkalotte braucht es Trägerkomponenten und Korrekturringe in unterschiedlichen Dimensionen.

## Patentansprüche

1. Modular aufgebaute Segmentprothese zum teilweisen Ersatz des Femurkopfs und seiner knorpeligen Oberfläche im Falle einer ossären, kartilaginären oder osteokartilaginären Läsion nicht näher definierter Ätiologie und Pathogenese, **bestehend** aus einer Trägerkomponente (3), einer Prothesenkalotte (1) und mindestens einem zwischen Trägerkomponente und Prothesenkalotte eingebrachten konischen Korrekturring mit unterschiedlicher Ausformung (4, 5) wobei die Modularität der Segmentprothese durch konische Steckverbindungen (8, 13) zwischen allen Prothesenkomponenten sichergestellt ist **dadurch gekennzeichnet, dass** die Ausformung, das Material und die Oberflächenbeschaffenheit der Trägerkomponente für eine unzementierte oder zementierte Implantation ausgelegt ist, dass die Prothesenkalotte (1) so ausgebildet und dimensioniert ist, dass ein Kugelsegment mit einem Öffnungswinkel zwischen 90° und 120° und einem Femurkopfdurchmesser zwischen 38 mm und 60 mm prothetisch ersetzbar ist und die zugehörigen Prothesenkomponenten in im Vergleich zum ursprünglichen Femurkopfdurchmesser adaptierter unterschiedlicher Dimension hergestellt sind.

2. Modular aufgebaute Segmentprothese zum teilweisen Ersatz des Femurkopfs nach Anspruch 1, **dadurch gekennzeichnet, dass** die Trägerkomponente (3) topfförmig ausgebildet ist und die Aussenseite (7) und die Innenseite (6) des Mantels konisch ausgeformt sind, wobei der Konuswinkel des Aussenkonus zwischen 5° und 40°, der des Innenkonus zwischen 5° und 10° beträgt, dass Aussen- und Innenkonus koaxial zueinander ausgerichtet sind.

3. Modular aufgebaute Segmentprothese zum teilweisen Ersatz des Femurkopfs nach einem der Ansprüche 1 oder 2, **dadurch gekennzeichnet, dass** die Trägerkomponente (3) topfförmig, einen konischen Mantel aufweisend ausgeformt ist, um für die unzementierte Implantation eine Primärstabilität des Prothesenteils gegenüber dem Prothesenlager durch radiäre Vorspannung des Knochens zu erzielen durch Einpressen der Trägerkomponente (3) in die konische Ausfräsung des Prothesenlagers, wobei die Einpresskraft zum Setzen des Prothesenträgers indirekt über einen Drehmomentschlüssel oder direkt mit einer Lastmessdose bestimmbar ist, um die Druck- und Zugfestigkeit des Knochens als kritische Grösse nicht zu überschreiten.

4. Modular aufgebaute Segmentprothese zum teilweisen Ersatz des Femurkopfs nach einem der Ansprüche 1, 2 oder 3, **dadurch gekennzeichnet, dass** für die unzementierte Implantation die Trägerkomponente (3) aus Titan oder einer Titanlegierung besteht mit einer aufgerauhten äusseren Oberfläche, z.B. sandgestrahlt, Titanplasma beschichtet, oder mit Titangittemetz versehen, zur Verbesserung der Osteointegration und zur Erhöhung der Sekundärstabilität.

5. Modular aufgebaute Segmentprothese zum teilweisen Ersatz des Femurkopfs nach einem der Ansprüche 1, 2, 3, oder 4, **dadurch gekennzeichnet, dass** für die unzementierte Implantation die Trägerkomponente (3) durch sich im Boden der Trägerkomponente winkelstabil verblockende Fixationsschrauben (9) fixierbar ist.

6. Modular aufgebaute Segmentprothese zum teilweisen Ersatz des Femurkopfs nach Anspruch 5, **dadurch gekennzeichnet, dass** die Fixationsschrauben (9) einen sich im Boden der Trägerkomponente winkelstabil verblockenden Schraubenkopf aufweisen, dies entweder durch eine konische Ausformung des Schraubenkopfs mit entsprechendem Gegenkonus im Bereich des Bodens der Trägerkomponente (8), oder durch ein konisches Gewinde im Bereich des Schraubenkopfs (10), welches in einem entsprechend konischen Gegengewinde des Bodens der Trägerkomponente (3) einschraubbar ist.

7. Modular aufgebaute Segmentprothese zum teilweisen Ersatz des Femurkopfs nach Anspruch 1, **dadurch gekennzeichnet, dass** für die unzementierte Implantation die Trägerkomponente (3) als Alternative zur Pressfitfixation nach einem der vorhergehenden Ansprüche eine Aussenmantelfläche mit einem selbstschneidenden Zwei- oder Dreifachgewinde aufweist zum Einschrauben der Trägerkomponente direkt in die konische Ausfräsung des Prothesenlagers.

8. Modular aufgebaute Segmentprothese zum teilweisen Ersatz des Femurkopfs nach Anspruch 1, **dadurch gekennzeichnet, dass** für die zementierte Implantation die Trägerkomponente (3) aus rostfreiem Stahl mit einer glatten äusseren Oberfläche besteht.

9. Modular aufgebaute Segmentprothese zum teilweisen Ersatz des Femurkopfs nach Anspruch 1, **dadurch gekennzeichnet, dass** sie einen äusseren und einen inneren Korrekturring (4, 5) aufweist, wobei beim äusseren Korrekturring (4) sowohl die Aussenseite als auch die Innenseite konisch ausgeformt sind mit einem Konuswinkel zwischen 5° und 10°, dass Aussen- und Innenkonus koaxial oder je nach notwendiger Winkelkorrektur exzentrisch und desaxiert (4a) zueinander ausgerichtet sind, wobei der Schnittpunkt der Längsachsen von Innenkonus und Aussenkonus entweder auf Höhe der Oberfläche der Prothesenkalotte (12), auf der Höhe des Oberrandes (13) der Trägerkomponente (3) oder irgendwo dazwischen liegt.

10. Modular aufgebaute Segmentprothese zum teilweisen Ersatz des Femurkopfs nach Anspruch 1, **dadurch gekennzeichnet, dass** sie einen äusseren und einen inneren Korrekturring (4, 5) aufweist, wobei beim inneren Korrekturring (5) sowohl die Aussenseite als auch die Innenseite konisch ausgeformt sind mit einem Konuswinkel zwischen 5° und 10°, dass Aussen- und Innenkonus koaxial zueinander ausgerichtet sind und dass die Wand des doppelt konischen Korrekturrings in unterschiedlicher Wanddicke (5a, 5b) gefertigt ist, um eine Höhenkorrektur der Prothesenkalotte bei fix implantierter Trägerkomponente zu erlauben.

11. Modular aufgebaute Segmentprothese zum teilweisen Ersatz des Femurkopfs nach Anspruch 1, **dadurch gekennzeichnet, dass** alternativ durch den äusseren Korrekturring (4) die Höhenkorrektur und durch den inneren Korrekturring (5) die Winkelkorrektur möglich ist oder dass ein Korrekturring vorhanden ist, mit welchem Korrekturring simultan eine Höhenkorrektur und eine Winkelkorrektur zum präzisen Positionieren der Prothesenkalotte gegenüber der fix montierten Trägerkomponente erreichbar ist.

12. Modular aufgebaute Segmentprothese zum teilweisen Ersatz des Femurkopfs nach einem der Ansprüche 1 oder 9, **dadurch gekennzeichnet, dass** die Prothesenkalotte (1) einen nach aussen weisenden Konus (2) mit einem Konuswinkel von zwischen 5° und 10° aufweist zur Verbindung mit dem inneren konischen Korrekturring (5).

13. Modular aufgebaute Segmentprothese zum teilweisen Ersatz des Femurkopfs nach Anspruch 1, **dadurch gekennzeichnet, dass** die Prothesenkalotte (1) im Bereich des Kalottenrandes zirkulär einen orthograden Verlauf zur Kalottenoberfläche aufweist.

14. Modular aufgebaute Segmentprothese zum teilweisen Ersatz des Femurkopfs nach Anspruch 1, **dadurch gekennzeichnet, dass** die Prothesenkalotte (1) aus einem Metall, einer Metalllegierung, einem keramischen Material, einem Kunststoff, einer Metall-Kunststoff-Verbindung oder einer Keramik-Kunststoff-Verbindung besteht mit folgenden Eigenschaften: geringer Reibungskoeffizient gegenüber dem nativen Knorpel azetabulär, gute Benetzbarkeit, geringes Abriebverhalten.

15. Modular aufgebaute Segmentprothese zum teilweisen Ersatz des Femurkopfs nach Anspruch 1, **dadurch gekennzeichnet, dass** die Prothesenkalotte (1) aus einem biokompatiblen Material, z.B. Titan, besteht, welches sich dazu eignet, als Oberfläche für eine direkt im Labor auf die Prothesenkalotte gezüchtete Knorpelzellschicht, respektive Knorpelgewebeschicht zu dienen, und welche als artifiziell-biologischer Verbund in die Trägerkomponente, respektive den konischen Korrekturring einsetzbar ist.

## Claims

1. Modular femoral head-segment prosthesis for the partial replacement of a femoral head segment and its corresponding cartilaginous surface in case of an osseous, cartilaginous, or osteocartilaginous lesion of undefined aetiology and pathogenesis, comprising a carrier component (3), a prosthetic calvaria (1), and at least one conical corrective ring in between of different size or shape (4, 5), the prosthesis being modular due to the fact that the different prosthetic components are combinable due to fitting cones (8, 13) between each of the components, wherein said, that the shape, the material, and the surface structure of the carrier component allows an implantation with or without bone cement, that the prosthetic calvaria (1) is shaped and dimensioned in such a way, that a spherical segment of the femoral head with an opening angle between 90° and 120° and a femoral head diameter between 38 mm and 60 mm can be replaced by the prosthesis, and that the corresponding prosthetic components are manufactured in adapted dimensions respecting the original diameter of the femoral head.

2. Modular femoral head-segment prosthesis for the partial replacement of a femoral head segment of claim 1, wherein the carrier component (3) has a cup-like shape with a conically shaped outer wall (7) and a conically shaped inner wall (6), the outer wall having an angle of the cone between 5° and 40°, the inner wall an angle of the cone between 5° and 10°, and both cones being coaxially aligned.

3. Modular femoral head-segment prosthesis for the partial replacement of a femoral head segment of claim 1 or 2, wherein the carrier component (3) with its cup-like shape has a conically shaped outer wall to be pressed inside a corresponding conical recess in the bone of the femoral head leading to high primary stability due to a radial prestress of the bone, whereby the force of insertion is measured indirectly by the means of a torque measuring device or directly by means of a load cell to enable the insertion of the carrier component without exceeding the compressive or tensile strength of the bone tissue.

4. Modular femoral head-segment prosthesis for the partial replacement of a femoral head segment of claim 1, 2 or 3, wherein the carrier component (3) is made of titanium, a titanium alloy with a rough outer surface, e.g. sand blasted, covered with a layer of titanium plasma, or a titanium mesh, able to improve the osteointegration and thus enhance the secondary stability of the prosthetic component in case of implantation without bone cement.

5. Modular femoral head-segment prosthesis for the partial replacement of a femoral head segment of claim 1 to 4, wherein the carrier component (3) can be stabilized additionally with locked fixation screws providing angular stability (9) inserted in the bottom of the carrier component in case of implantation without bone cement.

6. Modular femoral head-segment prosthesis for the partial replacement of a femoral head segment of claim 5, wherein the fixation screws (9) have a screw head which can be locked in the bottom of the carrier component providing angular stability of the screw, whereby the locking mechanism can be either a conically shaped screw head which engages in a corresponding counter-cone (8) in the bottom of the carrier component or a conically shaped thread on the screw head (10) engaging in a corresponding conically shaped counter-thread in the bottom of the carrier component (3).

7. Modular femoral head-segment prosthesis for the partial replacement of a femoral head segment of claim 1, wherein as an alternative anchorage concept to press-fit fixation, the outer wall of the carrier component carries a self-tapping double or triple thread which can be screwed into the prepared corresponding conical recess of the femoral head.

8. Modular femoral head-segment prosthesis for the partial replacement of a femoral head segment of claim 1, wherein the carrier component (3) is made of stainless steel with a polished surface an its outer surface in case of implantation with bone cement.

9. Modular femoral head-segment prosthesis for the partial replacement of a femoral head segment of claim 1, wherein the prosthetic system comprises an external and an internal corrective ring (4, 5), whereby the external corrective ring (4) is conically shaped on its external and its internal surface with an angle of the cone between 5° and 10°, both cones either coaxially aligned, or depending on an angular correction needed also eccentrically, or not coaxially aligned (4a), the intersection of the axes being located either at the level of the prosthetic calvaria (12), the upper border (13) of the carrier component (3), or in between.

10. Modular femoral head-segment prosthesis for the partial replacement of a femoral head segment of claim 1, wherein the prosthetic system comprises an external and an internal corrective ring (4, 5), whereby the internal corrective ring (5) is conically shaped on its external and internal surface with an angle of the cone between 5° and 10°, both cones coaxially aligned, the ring manufactured with different thicknesses of its wall (5a, 5b) to allow precise vertical positioning of the prosthetic calvaria with respect to the remaining intact femoral head cartilage and the implanted carrier component.

11. Modular femoral head-segment prosthesis for the partial replacement of a femoral head segment of claim 1, wherein as an alternative technical solution the external corrective ring (4) allows the vertical alignment and the internal corrective ring (5) the angular alignment, or that both corrections are possible with one only corrective ring allowing the simultaneous vertical and angular alignment of the prosthetic calvaria with respect to the remaining intact femoral head cartilage and to the implanted carrier component.

12. Modular femoral head-segment prosthesis for the partial replacement of a femoral head segment of claim 1 or 9, wherein the prosthetic calvaria (1) carries a protruding external cone (2) with an angle of the cone between 5° and 10° fitting into the corresponding cone of the internal corrective ring (5).

13. Modular femoral head-segment prosthesis for the partial replacement of a femoral head segment of claim 1, wherein the prosthetic calvaria (1) has on its periphery a free border being shaped perpendicular to the surface of the remaining intact cartilage of the femoral head.

14. Modular femoral head-segment prosthesis for the partial replacement of a femoral head segment of claim 1, wherein the material of the prosthetic calvaria (1) can be a metal, a metal alloy, a ceramic material, a polymer, a combination of metal and polymer or ceramic and polymer with the following properties: low coefficient of friction with the native cartilage of the cotyloid fossa, good lubrification, and low wear due to friction.

15. Modular femoral head-segment prosthesis for the partial replacement of a femoral head segment of claim 1, wherein the prosthetic calvaria (1) is produced of a biocompatible material suitable to serve as a carrier for the direct culture of cartilage cells or tissue layers in a laboratory, implantable as an artificial-biologic composite into the carrier component, or the conical corrective ring, respectively.

## Revendications

1. Prothèse partielle modulaire pour le remplacement d'un segment de la tête fémorale en cas d'une lésion cartilagineuse, osseuse ou ostéo-cartilagineuse d'une étiologie et pathogenèse inconnu, comprenant un embase (3), une calotte prothétique (1) et au moins un anneau conique de correction placé entre les deux composantes principales de design différent (4, 5), ensemble prothétique modulaire par des fixation conique entre toutes les composantes de prothèse (8, 13), **caractérisée par le fait que** la forme, le matériel et la structure de la surface de l'embase (3) est implantable avec ou sans ciment à os, que la calotte prothétique (1) est de forme et dimension permettant de remplacer un segment de sphère à un angle d'ouverture entre 90° et 120° et de remplacer une partie d'une tête fémorale d'un diamètre entre 38 mm et 60 mm et que les composantes de la prothèse sont manufacturées en différentes tailles de manière adaptée à la forme et à la taille originale de la tête fémorale.

2. Prothèse partielle modulaire pour le remplacement d'un segment de la tête fémorale selon la revendication 1, **caractérisée par le fait que** l'embase (3) a une forme de pot dont le manteau externe (7) et interne (6) est conique, que l'angle du cône du manteau externe est entre 5° et 40°, du manteau interne entre 5° et 10°, et que les deux cônes ont une orientation coaxiale.

3. Prothèse partielle modulaire pour le remplacement d'un segment de la tête fémorale selon la revendication 1 ou 2, **caractérisée par le fait que** l'embase (3) d'une forme de pot avec le manteau externe conique permets l'implantation sans ciment à os en pressant l'embase (3) dans la cavité osseuse d'une forme conique correspondante créant une haute stabilité primaire par une précontrainte radiale entre la composante prothétique et l'os en faisant spécifiquement attention au fait de ne pas dépasser la solidité osseuse en utilisant durant l'insertion soit une clé dynamométrique ou un instrument capable de mesurer directement la précontrainte radiale.

4. Prothèse partielle modulaire pour le remplacement d'un segment de la tête fémorale selon la revendication 1, 2 ou 3, **caractérisée par le fait que** l'embase (3) est construit en titane ou en un alliage de titane à surface rugueuse, par exemple sablée, couverte de plasma de titane ou d'un filet de titane en cas d'implantation sans ciment afin d'améliorer l'ostéointegration et aussi la stabilité secondaire.

5. Prothèse partielle modulaire pour le remplacement d'un segment de la tête fémorale selon la revendication 1 à 4, **caractérisée par le fait que** l'embase (3) peut être fixé additionnellement par de vis verrouillées à un angle fixe (9) dans le fond de l'embase en cas d'implantation non cimentée.

6. Prothèse partielle modulaire pour le remplacement d'un segment de la tête fémorale selon la revendication 5, **caractérisée par le fait que** les vis de verrouillage (9) portent une tête de vis conique non filetée ou filetée (10) se bloquant à un angle fixe dans des trous coniques non filetés ou filetés correspondants situés dans le fond de l'embase (8).

7. Prothèse partielle modulaire pour le remplacement d'un segment de la tête fémorale selon la revendication 1, **caractérisée par le fait que** l'embase (3) peut comporter comme système d'ancrage alternatif à la précontrainte radiale (press-fit), selon un des revendications antérieures des pas de vis doubles ou triples auto-taraudants permettant le vissage direct dans le fraisage conique de l'os de la tête du fémur.

8. Prothèse partielle modulaire pour le remplacement d'un segment de la tête fémorale selon la revendication 1, **caractérisée par le fait que** l'embase (3) est réalisée en acier avec une surface lisse à son extérieur en cas d'implantation cimentée.

9. Prothèse partielle modulaire pour le remplacement d'un segment de la tête fémorale selon la revendication 1, **caractérisée par le fait que** des anneaux de correction externe et interne (4, 5) sont à disposition, que l'anneau de correction externe (4) a une forme conique à son manteau externe et interne avec un angle de cône entre 5° et 10°, que les deux anneau de correction sont alignés soit de manière coaxiale, soit de manière excentrique ou désaxée (4a) et que la section entre les deux axes des anneaux soit située soit au niveau de la surface de la calotte prothétique (12) soit au niveau de bord supérieur (13) de l'embase (3) soit entre les deux positions décrites.

10. Prothèse partielle modulaire pour le remplacement d'un segment de la tête fémorale selon la revendication 1, **caractérisée par le fait que** des anneaux de correction externe et interne (4, 5) sont à disposition, que l'anneau de correction interne (5) a une forme conique à son manteau externe et interne avec un angle de cône d'entre 5° et 10°, que les deux cônes sont alignés dans la même axe et que de différentes épaisseurs du parois (5a, 5b) sont disponibles permettant une correction de la hauteur du positionnement de la calotte prothétique par rapport à l'embase déjà implantée.

11. Prothèse partielle modulaire pour le remplacement d'un segment de la tête fémorale selon la revendication 1, **caractérisée par le fait que** comme solution technique alternative l'anneau de correction externe (4) est capable de corriger la hauteur et que l'anneau de correction interne (5) est capable de corriger l'angle de positionnement de la calotte prothétique, ou que les deux fonctions de correction (hauteur et angle) sont disponibles simultanément avec un seul anneau de correction permettant le positionnement précis de la calotte prothétique par rapport à l'embase déjà implantée.

12. Prothèse partielle modulaire pour le remplacement d'un segment de la tête fémorale selon la revendication 1 ou 9, **caractérisée par le fait que** la calotte prothétique (1) porte dans sa partie distale un cône (2) avec un angle de cône d'entre 5° et 10° permettant son insertion dans l'anneau de correction interne (5).

13. Prothèse partielle modulaire pour le remplacement d'un segment de la tête fémorale selon la revendication 1, **caractérisée par le fait que** la calotte prothétique (1) est construite de manière que le bord libre proximal montre une orientation orthograde par rapport à la surface cartilagineuse.

14. Prothèse partielle modulaire pour le remplacement d'un segment de la tête fémorale selon la revendication 1, **caractérisée par le fait que** la calotte prothétique (1) est faite soit d'un métal, d'un alliage de métaux, d'un céramique, d'un polymère ou d'une combinaison de métal et de polymère ou d'un céramique et de polymère avec les attributs les suivants : coefficient de friction bas avec la surface cartilagineuse du cotyle, bonne lubrification et bonne résistance à l'usure.

15. Prothèse partielle modulaire pour le remplacement d'un segment de la tête fémorale selon la revendication 1, **caractérisée par le fait que** la calotte prothétique (1) est faite d'un matériel de haute biocompatibilité, par exemple titane, et que cette calotte est utilisable aussi dans un laboratoire spécifique où une couche de cellules cartilagineuses ou de tissu cartilagineux peut être cultivé permettant son insertion comme structure composée (artificielle et biologique) dans l'embase, respectivement dans l'anneau de correction interne.
